# FASCICULE DE BREVET EUROPEEN

(11) **EP 3 426 164 B1**
(45) Date de publication et mention de la délivrance du brevet: **12.05.2021**
(21) Numéro de dépôt: 17711780.1
(22) Date de dépôt: 09.03.2017
(51) Int. Cl.: A61B 17/04

(54) **ANCRE À USAGE MÉDICAL, DESTINÉE À ÊTRE INSÉRÉE DANS UNE PAROI OSSEUSE**
ANKER FÜR MEDIZINISCHE ZWECKE ZUM EINSETZEN IN EINE KNOCHENWAND
ANCHOR FOR MEDICAL USE, TO BE INSERTED INTO A BONY WALL

(30) Priorité: 10.03.2016 FR 1652026; 17.10.2016 FR 1660055
(43) Date de publication de la demande: 16.01.2019
(60) Demande divisionnaire: 21163604.8
(73) Titulaire: Dedienne Sante, 34130 Mauguio (FR); Chaouat, Gilles, 77330 Ozoir La Ferrière (FR); Decrette, Edouard, 27930 Gauciel (FR); Cohen, Gilles, 75017 Paris (FR); Fischer, Marc, 92100 Boulogne Billancourt (FR); Zeitoun, Jean-Marc, 75006 Paris (FR)
(72) Inventeur: CHAOUAT, Gilles, 77330 Ozoir La Ferrière (FR); DECRETTE, Edouard, 27930 Gauciel (FR); COHEN, Gilles, 75017 Paris (FR); FISCHER, Marc, 92100 Boulogne Billancourt (FR); ZEITOUN, Jean-Marc, 75006 Paris (FR); LEFIEF, Damien, 30320 Poulx (FR)
(74) Mandataire: LLR
(86) Numéro de dépôt international: PCT/IB2017/051396
(87) Numéro de publication internationale: WO 2017/153949

(56) Documents cités:
- EP-A1- 1 917 917
- EP-A2- 1 588 666
- FR-A1- 2 682 867
- US-A1- 2005 245 932
- US-A1- 2006 100 630
- US-A1- 2007 005 068
- US-A1- 2008 051 836
- US-A1- 2012 059 429

## Description

La présente invention concerne une ancre à usage médical, destinée à être insérée dans une paroi osseuse.

Cette ancre permet de fixer un ou plusieurs fils de suture à un os pour, notamment réinsérer un tendon ou un ligament sur un os ; elle est particulièrement destinée à permettre de réinsérer, sur un humérus, un ou plusieurs des tendons de la coiffe des rotateurs de l'articulation de l'épaule.

Il est bien connu, dans diverses indications, de refixer un tendon ou un ligament sur un os au moyen d'une ancre insérée dans la paroi de cet os, cette ancre étant reliée à un ou plusieurs fils de suture qui permettent de suturer le tendon ou ligament à l'os.

Pour relier le fil de suture à une ancre, il est connu de prévoir un passage transversal aménagé au travers de l'ancre, au travers duquel le fil de suture est engagé. Ce type d'assemblage a pour inconvénient de générer des détériorations au fil lors de la mise en place de l'ancre, dues aux frottements entre l'ancre et la paroi osseuse. Ces détériorations peuvent conduire à une rupture de ce fil.

Il est également connu de prévoir un anneau proximal sur une ancre, auquel le fil de suture est noué. Ce type d'ancre présente un risque notable de rupture du fil au niveau du nœud.

Pour remédier à cet inconvénient, il a été conçu des ancres dites "*knotless*"*,* dans lesquelles le fil est retenu sur l'ancre par pincement ou coincement. Les ancres existantes n'excluent cependant pas tout risque de détérioration du fil lors de la mise en place de l'ancre, particulièrement lorsque cette dernière est vissée, ou des risques de sectionnement du fil au niveau de son point de liaison à l'ancre.

La présente invention vise à remédier à cet inconvénient essentiel.

Par ailleurs, un certain nombre des ancres existantes présente une résistance à l'arrachement relativement incertaine. Ce problème se pose en particulier pour des ancres destinées à la réinsertion des tendons de la coiffe des rotateurs d'une articulation de l'épaule, compte tenu de la tension notable exercée sur le ou les fils de suture, et donc sur la ou les ancres, par ces tendons lors du mouvement de l'articulation.

La présente invention vise également à remédier à cet inconvénient.

L'objectif principal de l'invention est donc de fournir une ancre à laquelle un ou plusieurs fils de suture peuvent être parfaitement fixés et retenus, avec un risque réduit de cisaillement ou de rupture de ce ou ces fils.

Un autre objectif de l'invention est de fournir une ancre apte à être parfaitement fixée à une paroi osseuse.

Les publications de demandes de brevet N° US 2008/051836 A1, US 2005/245932 A1, EP 1 917 917 A1, FR 2 682 867 A1, US 2007/005068 A1 et US 2012/059429 A1 divulguent divers dispositifs selon la technique antérieure, .

L'ancre concernée comprend, de manière connue, les caractéristiques mentionnées dans la partie pré-caractérisante de la revendication 1 annexée. L'invention est définie par la partie caractérisante de cette revendication 1.

Il sera compris que les termes "proximal" et "distal" utilisés ci-dessus et dans l'ensemble de la présente description sont à considérer, de façon classique, par rapport au sens d'insertion de l'ancre dans une paroi osseuse, le terme "proximal" désignant un emplacement plus proche d'un praticien par rapport à ce sens d'insertion et "distal" désignant un emplacement plus éloigné de ce praticien par rapport à ce même sens d'insertion.

L'invention fournit une ancre dans laquelle les brins du ou des fils de suture se trouvent à l'intérieur de l'ancre lors de la mise en place de l'ancre et sont donc préservés de détériorations qui, autrement, seraient générées par les parois de l'ancre et de l'os lors de cette mise en place. La pièce extérieure peut alors comprendre extérieurement une structure permettant un ancrage renforcé à l'os ; il peut s'agir de nervures d'ancrage ayant une hauteur importante, et/ou d'une section transversale non circulaire de la pièce extérieure, notamment oblongue, faisant que la pièce extérieure est apte, dans une première position angulaire, à être engagée dans un trou de réception aménagé dans l'os, puis à être pivotée en force dans ce trou, jusqu'à une autre position angulaire, décalée de l'ordre de quatre-vingt-dix degrés par rapport à ladite première position angulaire, de façon à coincer l'ancre dans le trou.

Avant mise en place de l'ancre, la pièce intérieure peut ne pas être assemblée à la pièce extérieure, faisant que chaque fil de suture peut être facilement engagé sur l'ancre.

Une fois l'ancre mise en place sur l'os, une traction exercée sur les brins du ou des fils de suture permet de déplacer la pièce intérieure par rapport à la pièce extérieure vers ladite position proximale de blocage, réalisant un blocage de ce ou ces fils par coincement entre lesdites première et deuxième surfaces de coincement.

Ces surfaces de coincement, disposées longitudinalement, permettent d'exercer une pression sur une portion relativement importante du ou des fils, assurant un parfait coincement de ce ou ces fils, sans risque de cisaillement.

Ainsi les deux brins de chaque fil de suture sont coincés dans l'ancre par deux paires de surfaces de coincement, une située avant le passage du ou des fils au travers dudit conduit transversal, et l'autre située après ce passage.

De préférence, au moins une desdites première et deuxième surfaces de coincement est inclinée par rapport à l'axe longitudinal de l'ancre.

Le coincement du ou des fils de suture est ainsi réalisé par réduction progressive, lors du déplacement de ladite pièce intérieure vers ladite position proximale de blocage, de l'espace existant entre lesdites première et deuxième surface de coincement.

**Selon** l'invention, ladite pièce extérieure forme au moins une première surface d'engagement, distincte de ladite première surface de coincement, et ladite pièce intérieure forme au moins une deuxième surface d'engagement, distincte de ladite deuxième surface de coincement, ces surfaces d'engagement respectives venant en engagement mutuel dans ladite position proximale de blocage et réalisant une immobilisation de ladite pièce intérieure par rapport à ladite pièce extérieure dans cette position.

Un risque de retour de ladite pièce intérieure dans ladite position distale de non blocage est ainsi éliminé.

De préférence, ces première et deuxième surfaces d'engagement sont inclinées par rapport à l'axe longitudinal de l'ancre de manière à être aptes à venir, lorsque la pièce intérieure est déplacée dans le sens proximal à l'intérieur de la pièce extérieure, dans un état de coincement mutuel qui détermine ladite position proximale de blocage, et lesdites surfaces de coincement respectives sont aménagées de telle sorte qu'il existe entre elles, dans cette position proximale de blocage, un espace dont l'épaisseur est de l'ordre de 10 à 40% du diamètre d'un fil de suture.

Les surfaces d'engagement sont ainsi aménagées de manière à ce que leur coincement mutuel intervienne avant que lesdites surfaces de coincement respectives réalisent un écrasement excessif du ou des fils de suture. Le risque d'un tel écrasement excessif est éliminé de cette façon.

Selon l'invention,
- ladite pièce extérieure et ladite pièce intérieure présentent des moyens respectifs de positionnement angulaire de ladite pièce intérieure dans ladite pièce extérieure, plaçant ledit conduit transversal de la pièce intérieure dans une position angulaire déterminée par rapport à la pièce extérieure ;
- ladite pièce extérieure présente une cavité conique femelle dans laquelle sont aménagés deux conduits diamétralement opposés, de passage des brins du ou des fils de suture destinés à être reliés à l'ancre ; ces conduits sont aménagés sur un diamètre qui est parallèle à l'axe dudit conduit transversal lorsque ladite pièce intérieure se trouve dans ladite position angulaire déterminée ; les parois délimitant ces conduits sur le côté radialement extérieur de la pièce extérieure forment lesdites premières surfaces de coincement ; les surfaces délimitant ladite cavité conique femelle entre ces conduits forment deux dites premières surfaces d'engagement ;
- ladite pièce intérieure présente une portion tronconique mâle de laquelle font saillie deux bossages diamétralement opposés ; ces bossages sont aménagés sur un diamètre parallèle à l'axe dudit conduit transversal ; ces bossages sont destinés à s'engager dans lesdits conduits lors du déplacement de ladite pièce intérieure de ladite position distale de non blocage à ladite position proximale de blocage, et à coincer les brins du ou des fils de suture entre eux et lesdites parois délimitant les conduits vers l'extérieur dans la direction radiale ; les bossages constituent lesdites deuxièmes surfaces de coincement, et les surfaces de ladite portion tronconique mâle situées en dehors de ces bossages forment deux dites deuxièmes surfaces d'engagement, destinées à venir en engagement avec lesdites premières surfaces d'engagement dans ladite position proximale de blocage.

Lesdits moyens respectifs de positionnement peuvent notamment être constitués par deux rainures longitudinales diamétralement opposées aménagées dans ladite pièce extérieure et par des nervures correspondantes aménagés sur la pièce intérieure, ces nervures formant des patins destinés à être reçus dans la glissière que forment les rainures.

Avantageusement, la pièce extérieure présente deux lumières longitudinales diamétralement opposées, situées en regard des ouvertures dudit conduit transversal et s'étendant sur l'ensemble du déplacement de ce conduit, depuis ladite position distale de non blocage jusqu'à ladite position proximale de blocage.

Ces lumières permettent le passage du ou des fils de part et d'autre de la pièce intérieure lorsque cette pièce intérieure a un diamètre extérieur ajusté au diamètre intérieur de la cavité que forme la pièce extérieure.

De préférence, la pièce intérieure présente deux saillies d'encliquetage aptes à venir s'encliqueter derrière les bords de la pièce extérieure délimitant les extrémités distales desdites lumières.

Un montage et une rétention facile de la pièce intérieure dans la pièce extérieure est ainsi obtenu, par encliquetage.

De préférence, ladite pièce intérieure présente une portion distale pointue, faisant saillie au-delà de la pièce extérieure, du côté distal, lorsque ladite pièce intérieure est dans ladite position distale de non blocage.

Cette extrémité distale pointue favorise l'insertion et la progression de l'ancre dans le trou de la paroi osseuse.

L'invention, telle qu'exposée ci-dessus, sera bien comprise, et d'autres caractéristiques et avantages de celle-ci apparaîtront, en référence au dessin schématique annexé, qui représente, à titre d'exemple non limitatif, une forme de réalisation préférée de l'ancre concernée.

La figure 1 en est une vue en perspective, avec une pièce intérieure qu'elle comprend dans une position sortie par rapport à une pièce extérieure qu'elle comprend également ;

la figure 2 en est une vue en coupe longitudinale dans un plan médian passant par l'axe d'un conduit transversal que forme ladite pièce intérieure, cette pièce intérieure étant sortie par rapport à ladite pièce extérieure ;

la figure 3 en est une vue similaire à la figure 2, selon un plan de coupe perpendiculaire à celui selon la figure 2 ;

la figure 4 en est une vue similaire à la figure 1, après mise en place de fils de suture et avec la pièce intérieure placée dans la pièce extérieure, cette pièce intérieure étant dans une position distale de non blocage des fils de suture ;

la figure 5 en est une vue similaire à la figure 4, selon un plan de coupe identique à celui selon la figure 2 ; cette figure 5 montre également une portion d'extrémité d'une barre et une portion d'une tige que comprend un instrument servant à la mise en place de l'ancre dans une paroi osseuse ; la portion d'extrémité de la barre est vue partiellement en coupe longitudinale et partiellement en extérieur ;

la figure 6 est une vue de l'ancre similaire à la figure 4, selon un plan de coupe identique à celui selon la figure 3 ;

la figure 7 en est une vue avec ladite pièce intérieure dans une position proximale de blocage des fils de suture, selon un plan de coupe identique à celui selon la figure 2 ; et

la figure 8 en est une similaire à la figure 7, selon un plan de coupe identique à celui selon la figure 3.

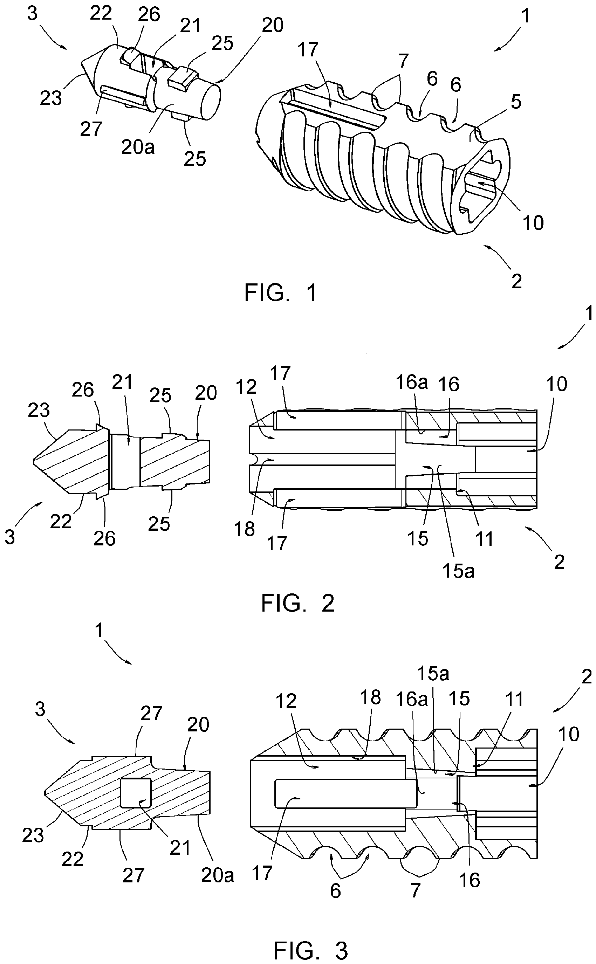

Les figures 1 à 3 représentent une ancre 1 à usage médical, destinée à être insérée dans une paroi osseuse et à fixer ainsi un ou plusieurs fils de suture 100 à cet os. Cette ancre 1 est particulièrement destinée à permettre de réinsérer sur un humérus un ou plusieurs des tendons de la coiffe des rotateurs de l'articulation de l'épaule.

L'ancre 1 comprend une pièce extérieure 2 et une pièce intérieure 3 destinées à être assemblées l'une à l'autre.

La pièce extérieure 2 est destinée à être fixée à l'os. À part une portion distale conique, elle a une section transversale en forme d'anneau aplati, faisant qu'elle présente deux méplats 5 sur deux côtés opposés et deux faces courbes entre ces méplats 5. Dans ces faces courbes, sont aménagées des séries de cannelures profondes 6, délimitant des séries de nervures 7 entre elles. La pièce 2 est apte, dans une première position angulaire d'insertion dans un trou aménagé dans la paroi osseuse, à être engagée dans ce trou, puis à être pivotée en force dans ce trou, jusqu'à une autre position angulaire, décalée de l'ordre de quatre-vingt-dix degrés par rapport à ladite première position angulaire, de façon à coincer l'ancre 1 dans le trou.

La pièce extérieure 2 forme une cavité longitudinale interne la traversant de part en part, délimitant une cavité proximale 10, une portion intermédiaire 11 et une cavité distale 12.

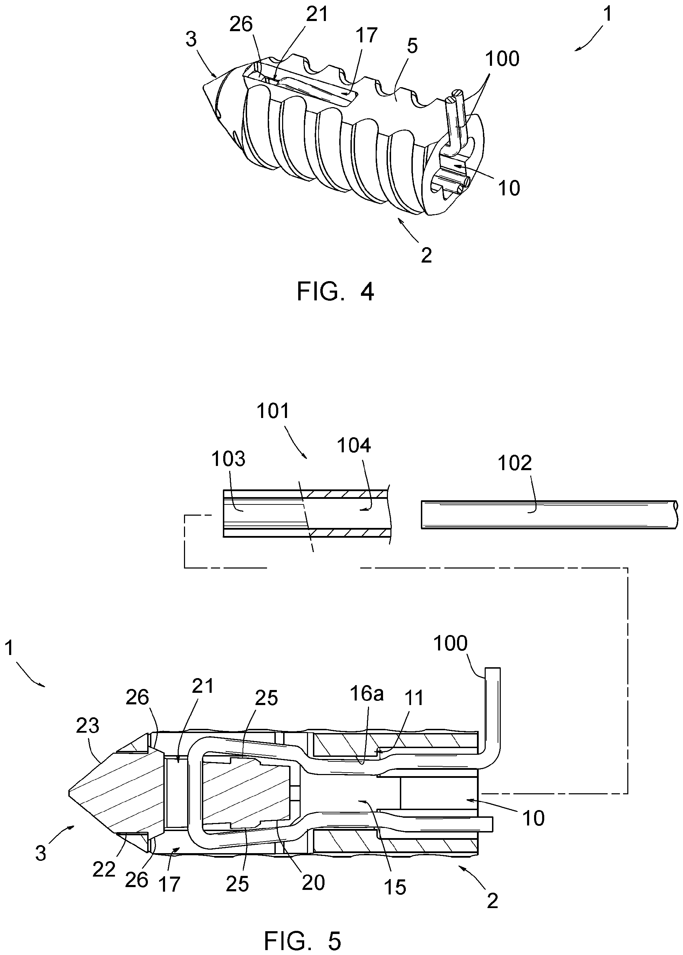

La cavité proximale 10 forme, le long des méplats 5, deux logements latéraux destinés à recevoir les fils de suture, comme visible sur la figure 4, et une cavité de montage centrale incluant deux rainures latérales, destinée à recevoir de façon ajustée l'extrémité distale de la barre 101, de section correspondante.

La portion intermédiaire 11 délimite une cavité conique femelle 15 ayant une pente faible, de l'ordre de 5 à 10 degrés, cette cavité 15 étant apte à recevoir en elle, avec coincement, une portion tronconique mâle 20 que comprend la pièce intérieure 3, décrite plus loin. Elle forme deux conduits 16 diamétralement opposés, situés en dessous des méplats 5 et situés dans le prolongement des deux logements latéraux précités que forme la cavité 10. Ces conduits 16 communiquent avec ces logements latéraux et avec la cavité distale 12, comme visible sur la figure 2 et permettent ainsi le passage au travers d'eux des brins des fils de suture 100, comme visible sur la figure 5.

Il apparaît sur la figure 2 que les parois 16a délimitant les fonds de ces conduits 16 sont parallèles à l'axe longitudinal de la pièce extérieure 2.

La cavité distale 12 communique avec l'extérieur de la pièce 2 par deux lumières 17, s'étendant au travers des méplats 5, et forme deux rainures 18 diamétralement opposées, s'étendant dans un plan diamétral perpendiculaire au plan dans lequel s'étendent les lumières 17.

La pièce intérieure 3 est destinée à être insérée et retenue dans la cavité distale 12 et dans la cavité femelle 15 de la pièce extérieure 2. Du côté proximal au côté distal, elle présente la portion tronconique mâle 20 précitée, une portion traversée par un conduit transversal 21, une portion d'encliquetage 22 et une portion distale 23.

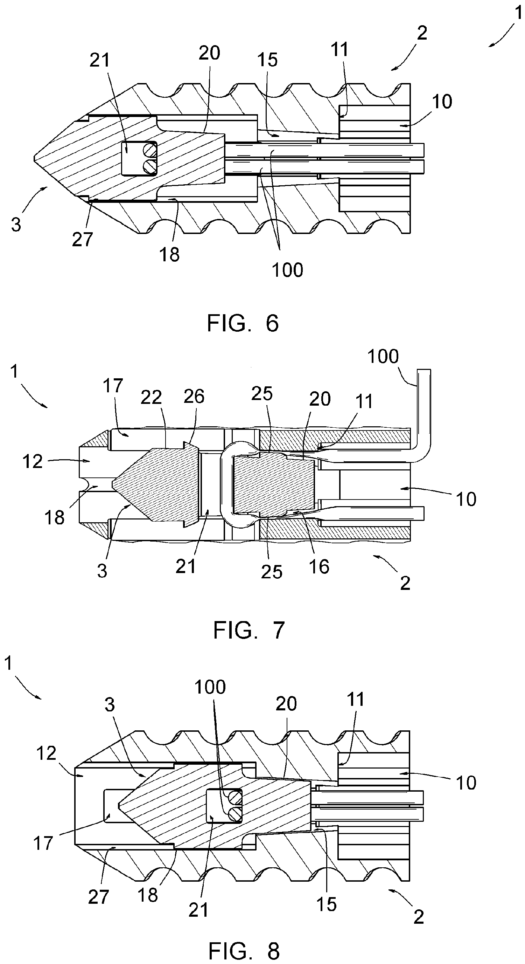

La portion tronconique mâle 20 a une pente correspondant à celle de ladite cavité femelle 15, de façon à être apte à être reçue avec coincement dans cette cavité. Elle forme une face proximale perpendiculaire à son axe longitudinal, contre laquelle la tige 102 coulissant dans la barre 101 est apte à venir en appui, comme décrit plus loin. Elle présente également deux bossages 25 diamétralement opposés, aménagés sur un diamètre de la portion 20 parallèle à l'axe du conduit transversal 21 ; ces bossages 25 sont destinés à s'engager dans lesdits conduits 16 lors du passage de la pièce intérieure 3 de la position distale de non blocage montrée sur les figures 5 et 6 à la position proximale de blocage montrée sur les figures 7 et 8, venant ainsi coincer les brins des fils de suture 100 entre eux et les parois 16a formant les fonds des conduits 16.

Le conduit transversal 21 traverse la pièce intérieure 3 de part en part et présente, dans l'exemple représenté une section transversale carrée. Il est destiné à être traversé par les fils de suture 100, comme visible sur les figures 5 à 8.

La portion d'encliquetage 22 présente deux saillies d'encliquetage 26 aptes à venir s'encliqueter derrière les bords de la pièce extérieure 2 délimitant les extrémités distales des lumières 17, comme visible sur la figure 5.

La portion distale conique 23 est pointue et sa paroi périphérique vient, dans la position distale de non blocage montrée sur la figure 5, dans le prolongement de la paroi de la portion distale conique de la pièce extérieure 2.

En outre, la pièce intérieure 3 forme, sur sa portion délimitant le conduit 21 et sur sa portion d'encliquetage 22, deux nervures allongées 27 diamétralement opposées, situées dans un plan perpendiculaire au plan dans lequel s'étend l'axe du conduit 21. Ces nervures 27 forment des patins destinés à être reçus dans la glissière que forment les rainures 18, comme visible sur les figures 6 et 8.

L'instrument partiellement représenté sur la figure 5 est conforme à celui décrit par la publication de demande de brevet français N° 16 52026. La barre 101 présente une section transversale carrée et, comme visible sur la figure 5, au niveau de sa portion d'extrémité distale, deux nervures latérales 103. Cette portion distale de la barre 101 est adaptée à être engagée dans la cavité de montage précitée que forme la cavité proximale 10, avec les nervures 103 reçues dans les deux rainures latérales précitées de cette cavité.

La barre 101 est percée longitudinalement d'un alésage 104 dans lequel la tige 102 précitée est engagée de façon coulissante. La tige 102 est mobile dans cet alésage 104 entre une position distale, dans laquelle elle vient en appui contre la face proximale de la pièce intérieure 3 alors que la barre 101 est insérée dans ladite cavité de montage, et une position proximale, dans laquelle elle est reculée vis-à-vis de cette face proximale. Dans sa position distale, la tige 102 maintient la pièce intérieure 3 dans la position distale de non blocage et, dans sa position proximale, ne fait pas obstacle au déplacement de la pièce intérieure 3 vers la position proximale de blocage visible sur les figures 7 et 8.

L'instrument comprend des moyens (non représentés, décrit dans la publication de demande de brevet français N° 16 52026) permettant de bloquer sélectivement la tige 102 vis-à-vis de la barre 101, dans ladite position distale de cette tige. Ces moyens peuvent notamment être sous la forme d'un tiroir porté transversalement par un manche solidaire de la barre 101, ce tiroir étant mobile transversalement à l'axe longitudinal de cette barre 101 ; le tiroir présente une portion de paroi pleine et une portion de paroi percée d'un trou de section supérieure à celui de la tige 102 ; dans une position de coulissement, ladite portion de paroi pleine de ce tiroir se trouve à proximité immédiate de l'extrémité proximale de la tige 102 et bloque ainsi tout recul de cette tige, et dans une autre position de coulissement de ce tiroir, le trou que forme ce tiroir se trouve en regard de la tige 102 et permet le recul de cette tige.

En pratique, les fils de suture 100 sont engagés au travers de la pièce extérieure 2, puis au travers du conduit transversal 21 puis à nouveau au travers de la pièce extérieure 2, et la pièce intérieure 3 est engagée dans la cavité distale 12, avec les nervures 27 dans les rainures 18, jusqu'à encliquetage des saillies 26 au-delà des bords de la pièce 2 délimitant les extrémités distales des lumières 17. L'ancre 1 est alors dans l'état visible sur les figures 4 à 6.

Pour réaliser l'insertion de l'ancre 1 dans une cavité osseuse, la portion d'extrémité libre de la barre 101 est engagée dans ladite cavité de montage et la tige 102 est bloquée dans sa position distale, maintenant ainsi la pièce intérieure 3 dans ladite position distale de non blocage visible sur les figures 5 et 6.

Une fois l'insertion de l'ancre 1 dans l'os réalisée, le recul de la tige 102 est débloqué, ce qui permet le déplacement de la pièce intérieure 3 vers la position proximale de blocage visible sur les figures 7 et 8 lorsqu'une traction est exercée sur les fils 100, en particulier lors de la remise en place des ligaments sur l'os. Dans cette position, les fils de suture 100 sont pincés entre les fonds 16a des conduits 16 et les faces radialement externes des bossages 25, ces fonds et faces constituant des surfaces de coincement respectives des fils 100.

Simultanément, les parties de la portion tronconique mâle 20 non en regard des conduits 16 viennent en engagement dans les parties de la cavité 15 situées en dehors des conduits 16, jusqu'à coincement, assurant une immobilisation de la pièce intérieure 3 par rapport à la pièce extérieure 2 dans cette position proximale.

L'invention fournit une ancre à usage médical présentant des avantages déterminants par rapport aux ancres homologues de la technique antérieure, notamment ceux de :
- préserver les brins du ou des fils de suture 100 des détériorations résultant de la mise en place de l'ancre, du fait que ces fils sont placés à l'intérieur de l'ancre 1 ;
- comprendre extérieurement une structure permettant le parfait ancrage de l'ancre 1 dans un os, notamment par coincement résultant d'un pivotement sur un quart de tour ;
- conserver une mise en place relativement facile du ou des fils de suture en elle, du fait de sa structure en deux pièces 2, 3 assemblables ;
- avoir un assemblage facile de ces deux pièces ;
- permettre d'exercer une pression sur une portion relativement importante du ou des fils de suture 100, assurant un parfait coincement de ce ou ces fils, sans risque de cisaillement.

L'ancre 1 a été décrite ci-dessus en référence à une forme de réalisation préférée ; il va de soi que cette description n'est en rien limitative.

## Revendications

1. Ancre (1) à usage médical, destinée à être insérée dans une paroi osseuse, comprenant deux pièces (2, 3) destinées à être assemblées l'une à l'autre, à savoir une pièce extérieure (2), destinée à être fixée à l'os, qui forme une cavité longitudinale interne à travers elle, et une pièce intérieure (3), destinée à être insérée et retenue dans ladite cavité de la pièce extérieure (2), qui forme un conduit transversal (21) la traversant de part en part ; au moins un fil de suture (100) étant destiné à être engagé dans ladite cavité par le côté proximal de la pièce extérieure (2), à être engagé au travers dudit conduit transversal (21) et à ressortir de ladite pièce extérieure (2) par ledit côté proximal; dans laquelle:
- la pièce extérieure (2) forme au moins une première surface de coincement (16a) disposée dans la direction longitudinale de la pièce extérieure (2), et la pièce intérieure (3) forme au moins une deuxième surface de coincement (25) située du côté proximal par rapport au conduit transversal (21), cette deuxième surface de coincement (25) étant disposée dans la direction longitudinale de la pièce intérieure (3) ;
- ledit au moins un fil de suture (100) est destiné à s'étendre le long de ladite première surface de coincement (16a) et le long de ladite deuxième surface de coincement (25) ;
- la pièce intérieure (3) est mobile par rapport à la pièce extérieure (2) entre une position distale de non blocage du fil de suture (100), dans laquelle ladite deuxième surface de coincement (25) est à une distance de ladite première surface de coincement (16a) ne réalisant aucun coincement de ce fil, et une position proximale de blocage du fil de suture (100), dans laquelle ladite deuxième surface de coincement (25) est à une distance de ladite première surface de coincement (16a) réalisant un coincement de ce fil ;
ladite pièce extérieure (2) forme au moins une première surface d'engagement (15a), distincte de ladite première surface de coincement (16a), ladite pièce intérieure (3) forme au moins une deuxième surface d'engagement (20a), distincte de ladite deuxième surface de coincement (25), ces surfaces d'engagement (15a, 20a) respectives venant en engagement mutuel dans ladite position proximale de blocage et réalisant une immobilisation de ladite pièce intérieure (3) par rapport à ladite pièce extérieure (2) dans cette position ;
**caractérisée en ce que** ladite pièce extérieure (2) et ladite pièce intérieure (3) présentent des moyens respectifs (18, 27) de positionnement angulaire de ladite pièce intérieure (3) dans ladite pièce extérieure (2), plaçant ledit conduit transversal (21) de la pièce intérieure (3) dans une position angulaire déterminée par rapport à la pièce extérieure (2) ;
ladite pièce extérieure (2) présente une cavité conique femelle (15) dans laquelle sont aménagés deux conduits (16) diamétralement opposés, de passage des brins du ou des fils de suture (100) destinés à être reliés à l'ancre (1) ; ces conduits (16) sont aménagés sur un diamètre qui est parallèle à l'axe dudit conduit transversal (21) lorsque ladite pièce intérieure (3) se trouve dans ladite position angulaire déterminée ; les parois délimitant ces conduits (16) sur le côté radialement extérieur de la pièce extérieure (2) forment lesdites premières surfaces de coincement (16a) ; les surfaces délimitant ladite cavité conique femelle (15) entre ces conduits (16) forment deux dites premières surfaces d'engagement (15a) ;
ladite pièce intérieure (3) présente une portion tronconique mâle (20) de laquelle font saillie deux bossages (25) diamétralement opposés ; ces bossages (25) sont aménagés sur un diamètre parallèle à l'axe dudit conduit transversal (21) ; ces bossages (25) sont destinés à s'engager dans lesdits conduits (16) lors du déplacement de ladite pièce intérieure (3) de ladite position distale de non blocage à ladite position proximale de blocage, et à coincer les brins du ou des fils de suture (100) entre eux et lesdites parois (16a) délimitant les conduits (16) vers l'extérieur dans la direction radiale ; les bossages (25) constituent lesdites deuxièmes surfaces de coincement, et les surfaces de ladite portion tronconique mâle (20) situées en dehors de ces bossages (25) forment deux dites deuxièmes surfaces d'engagement (20a), destinées à venir en engagement avec lesdites premières surfaces d'engagement (15a) dans ladite position proximale de blocage.

2. Ancre (1) selon la revendication 1, **caractérisée en ce qu'**au moins une desdites première et deuxième surfaces de coincement (16, 25) est inclinée par rapport à l'axe longitudinal de l'ancre (1).

3. Ancre (1) selon la revendication 1, **caractérisée en ce que** lesdites première et deuxième surfaces d'engagement (15a, 20a) sont inclinées par rapport à l'axe longitudinal de l'ancre (1) de manière à être aptes à venir, lorsque la pièce intérieure (3) est déplacée dans le sens proximal à l'intérieur de la pièce extérieure (2), dans un état de coincement mutuel qui détermine ladite position proximale de blocage, et **en ce que** lesdites surfaces de coincement (16a, 25) respectives sont aménagées de telle sorte qu'il existe entre elles, dans cette position proximale de blocage, un espace dont l'épaisseur est de l'ordre de 10 à 40% du diamètre d'un fil de suture (100).

4. Ancre (1) selon la revendication 1, **caractérisée en ce que** lesdits moyens respectifs de positionnement sont constitués par deux rainures longitudinales (18) diamétralement opposées aménagées dans ladite pièce extérieure (2) et par des nervures (27) correspondantes aménagés sur la pièce intérieure (3), ces nervures (27) formant des patins destinés à être reçus dans la glissière que forment les rainures (18).

5. Ancre (1) selon l'une des revendications 1 à 4, **caractérisée en ce que** la pièce extérieure (2) présente deux lumières longitudinales (17) diamétralement opposées, situées en regard des ouvertures dudit conduit transversal (21) et s'étendant sur l'ensemble du déplacement de ce conduit (21), depuis ladite position distale de non blocage jusqu'à ladite position proximale de blocage.

6. Ancre (1) selon la revendication 5, **caractérisée en ce que** la pièce intérieure (3) présente deux saillies d'encliquetage (26) aptes à venir s'encliqueter derrière les bords de la pièce extérieure (2) délimitant les extrémités distales desdites lumières (17).

7. Ancre (1) selon l'une des revendications 1 à 6, **caractérisée en ce que** ladite pièce intérieure (3) présente une portion distale (23) pointue, faisant saillie au-delà de la pièce extérieure (2), du côté distal, lorsque ladite pièce intérieure (3) est dans ladite position distale de non blocage.

## Patentansprüche

1. Verankerung (1) zur medizinischen Verwendung, bestimmt, um in eine Knochenwand eingesetzt zu werden, umfassend zwei Teile (2, 3), bestimmt, um miteinander verbunden zu werden, das heißt ein äußeres Teil (2), bestimmt, um an dem Knochen fixiert zu werden, das einen längsgerichteten internen Hohlraum durch es hindurch bildet, und ein inneres Teil (3), bestimmt, um in den Hohlraum des äußeren Teils (2), der einen Querkanal (21) bildet, der es von einer Seite zur anderen durchquert, eingesetzt und darin zurückgehalten zu werden; mindestens einen Nahtdraht (100), der bestimmt ist, um von der proximalen Seite des äußeren Teils (2) in dem Hohlraum in Eingriff gebracht zu werden, um durch den Querkanal (21) hindurch in Eingriff gebracht zu werden und aus dem äußeren Teil (2) durch die proximale Seite wieder herauszutreten, wobei:
- das äußere Teil (2) mindestens eine in der längsgerichteten Richtung des äußeren Teils (2) angeordnete erste Klemmfläche (16a) bildet und das innere Teil (3) mindestens eine zweite Klemmfläche (25) bildet, die bezogen auf den Querkanal (21) auf der proximalen Seite liegt, wobei diese zweite Klemmfläche (25) in der längsgerichteten Richtung des inneren Teils (3) angeordnet ist;
- der mindestens eine Nahtdraht (100) bestimmt ist, um sich entlang der ersten Klemmfläche (16a) und entlang der zweiten Klemmfläche (25) zu erstrecken;
- das innere Teil (3) bezogen auf das äußere Teil (2) zwischen einer distalen Position des Nichtblockierens des Nahtdrahts (100), in der die zweite Klemmfläche (25) in einem Abstand von der ersten Klemmfläche (16a) vorliegt, wobei kein Einklemmen dieses Drahts ausgeführt wird, und einer proximalen Position des Blockierens des Nahtdrahts (100) beweglich ist, in der die zweite Klemmfläche (25) in einem Abstand von der ersten Klemmfläche (16a) vorliegt, wobei ein Einklemmen dieses Drahts ausgeführt wird;
wobei das äußere Teil (2) mindestens eine von der ersten Klemmfläche (16a) verschiedene erste Eingriffsfläche (15a) bildet, und dadurch, das innere Teil (3) mindestens eine von der zweiten Klemmfläche (25) verschiedene zweite Eingriffsfläche (20a) bildet, wobei diese Eingriffsflächen (15a, 20a) in der proximalen Position des Blockierens jeweils in gegenseitigen Eingriff kommen und in dieser Position eine Immobilisierung des inneren Teils (3) bezogen auf das äußere Teil (2) ausführen, **dadurch gekennzeichnet, dass**
das äußere Teil (2) und das innere Teil (3) jeweilige Mittel (18, 27) zur Winkelpositionierung des inneren Teils (3) in dem äußeren Teil (2) aufweisen, die den Querkanal (21) des inneren Teils (3) in eine bezogen auf das äußere Teil (2) festgelegte Winkelposition platzieren;
das äußere Teil (2) einen konischen Aufnahmehohlraum (15) aufweist, in dem zwei diametral gegenüberliegende Kanäle (16) zum Durchgang von Strängen des oder der Nahtdrähte (100), bestimmt, um mit der Verankerung (1) verbunden zu werden, ausgebildet sind; wobei diese Kanäle (16) auf einem Durchmesser ausgebildet sind, der parallel zur Achse des Querkanals (21) ist, wenn das innere Teil (3) sich in der festgelegten Winkelposition befindet; wobei die diese Kanäle (16) auf der radial äußeren Seite des äußeren Teils (2) begrenzenden Wände die ersten Klemmflächen (16a) bilden; wobei die den konischen Aufnahmehohlraum (15) zwischen diesen Kanälen (16) begrenzenden Flächen zwei der ersten Eingriffsflächen (15a) bilden;
das innere Teil (3) einen kegelstumpfförmigen Steckabschnitt (20) aufweist, von dem zwei diametral gegenüberliegende Erhebungen (25) hervorstehen; wobei diese Erhebungen (25) auf einem Durchmesser parallel zur Achse des Querkanals (21) ausgebildet sind; wobei diese Erhebungen (25) bestimmt sind, um bei Verschiebung des inneren Teils (3) von der distalen Position des Nichtblockierens zur proximalen Position des Blockierens in die Kanäle (16) einzugreifen, und die Stränge des oder der Nahtdrähte (100) zwischen sich und den Wänden (16a), die die Kanäle (16) in der radialen Richtung nach Außen begrenzen, einzuklemmen; wobei die Erhebungen (25) die zweiten Klemmflächen ausmachen und die Flächen des kegelstumpfförmigen Steckabschnitts (20), die außerhalb dieser Erhebungen (25) liegen, zwei zweite Eingriffsflächen (20a) bilden, bestimmt, um mit den ersten Eingriffsflächen (15a) in der proximalen Position des Blockierens in Eingriff zu kommen.

2. Verankerung (1) nach Anspruch 1, **dadurch gekennzeichnet, dass** mindestens eine der ersten und zweiten Klemmflächen (16, 25) bezogen auf eine Längsachse der Verankerung (1) geneigt ist.

3. Verankerung (1) nach Anspruch 1, **dadurch gekennzeichnet, dass** die ersten und zweiten Eingriffsflächen (15a, 20a) bezogen auf die Längsachse der Verankerung (1) auf eine Weise geneigt sind, um geeignet zu sein, in einen Zustand des gegenseitigen Einklemmens zu kommen, der die proximale Position des Blockierens festlegt, wenn das innere Teile (3) in proximaler Richtung zum Inneren des äußeren Teils (2) bewegt wird, und dadurch, dass die jeweiligen Klemmflächen (16a, 25) derart ausgebildet sind, dass zwischen ihnen in der proximalen Position des Blockierens ein Raum existiert, von dem die Dicke in der Größenordnung von 10 bis 40 % des Durchmessers eines Nahtdrahts (100) liegt.

4. Verankerung (1) nach Anspruch 1, **dadurch gekennzeichnet, dass** die jeweiligen Mittel der Positionierung aus zwei diametral gegenüberliegenden längsgerichteten Aussparungen (18), ausgebildet in dem äußeren Teil (2) und durch entsprechende auf dem inneren Teil (3) ausgebildete Rippen (27) bestehen, wobei diese Rippen (27) Gleitstücke bilden, bestimmt, um in der Gleitschiene, die die Aussparungen (18) bilden, aufgenommen zu werden.

5. Verankerung (1) nach einem der Ansprüche 1 bis 4, **dadurch gekennzeichnet, dass** das äußere Teil (2) zwei längsgerichtete diametral gegenüberliegende Lumen (17) aufweist, die den Öffnungen des Querkanals (21) gegenüber liegen und sich ab der distalen Position des Nichtblockierens bis zu der proximalen Position des Blockierens auf dem gesamten Verfahrweg dieses Kanals (21) erstrecken.

6. Verankerung (1) nach Anspruch 5, **dadurch gekennzeichnet, dass** das innere Teil (3) zwei Rastvorsprünge (26) aufweist, geeignet, um hinter die Kanten des äußeren Teils (2), die die distalen Enden der Lumen (17) begrenzen, einzurasten.

7. Verankerung (1) nach einem der Ansprüche 1 bis 6, **dadurch gekennzeichnet, dass** das innere Teil (3) einen distalen spitzen Abschnitt (23) aufweist, der jenseits des äußeren Teils (2) von der distalen Seite hervorsteht, wenn das innere Teil (3) in der distalen Position des Nichtblockierens vorliegt.

## Claims

1. Anchor (1) for medical use, intended to be inserted into a bony wall, comprising two parts (2, 3) that are to be assembled, i.e., an outer part (2), intended to be fastened to the bone, which forms an inner longitudinal cavity through it, and an inner part (3), intended to be inserted and held in said cavity of the outer part (2), which forms a transverse conduit (21) passing all the way through it; at least one suture thread (100) being intended to be engaged in said cavity through the proximal side of the outer part (2), to be engaged through said transverse conduit (21) and to emerge from said outer part (2), through said proximal side; wherein:
- the outer part (2) forms at least one first jamming surface (16a) arranged in the longitudinal direction of the outer part (2) and the inner part (3) forms at least one second jamming surface (25) located on the proximal side relative to the transverse conduit that it comprises, this second jamming surface (25) being arranged in the longitudinal direction of the inner part (3) ;
said at least one suture thread (100) is intended to extend along said first jamming surface (16a) and along said second jamming surface (25);
the inner part (3) is movable relative to the outer part (2) between a distal position not blocking the suture thread (100), in which said second jamming surface (25) is at a distance from said first jamming surface (16a) not performing any jamming of this thread, and a proximal blocking position of the suture thread (100), in which said second jamming surface (25) is at a distance from said first jamming surface (16a) performing jamming of this thread.;
said outer part (2) forms at least a first engagement surface (15a), separate from said first jamming surface (16a), said inner part (3) forms at least one second engagement surface (20a), separate from said second jamming surface (25), these respective engagement surfaces (15a, 20a) mutually engaging in said proximal blocking position and immobilizing said inner part (3) relative to said outer part (2) in this position. ;
**characterized in that** said outer part (2) and said inner part (3) have respective means (18, 27) for angular positioning of said inner part (3) in said outer part (2), placing said transverse conduit (21) of the inner part (3) in a determined angular position relative to the outer part (2);
said outer part (2) has a female conical cavity (15) in which two diametrically opposite conduits (16) are arranged, for passage of the strands of the suture thread(s) (100) intended to be connected to the anchor (1); these conduits (16) are arranged over a diameter that is parallel to the axis of said transverse conduit when said inner part (3) is in said determined angular position; the walls delimiting these conduits (16) on the radially outer side of the outer part (2) forms said first jamming surfaces (16a); the surfaces delimiting said female conical cavity between these conduits (16) form two said first engagement surfaces (15a);
said inner part (3) has a male frustoconical portion (20) from which two diametrically opposite bosses (25) protrude; these bosses (25) are arranged over a diameter parallel to the axis of said transverse conduit (21); these bosses (25) are intended to engage in said conduits (16) during the movement of said inner part (3) from said distal nonblocking position to said proximal blocking position, and to jam the strands of the suture threads (100) between them and said walls (16a) delimiting the conduits (16) toward the outside in the radial direction; the bosses (25) make up said second jamming surfaces, and the surfaces of said male frustoconical portion (20) located outside these bosses (25) form two said second engagement surfaces (20a), intended to engage with said first engagement surfaces (15a) in said proximal blocking position..

2. The anchor (1) according to claim 1, **characterized in that** said outer part (2) comprises two said first jamming surfaces (16a), diametrically opposite, and said inner part (3) comprises two corresponding said second jamming surfaces (25).

3. The anchor (1) according to claim 4, **characterized in that** said first and second engagement surfaces (15a, 20a) are inclined relative to the longitudinal axis of the anchor so as to be able, when the inner part (3) is moved in the proximal direction inside the outer part (2), to enter a mutual jamming state that determines said proximal blocking position, and said respective jamming surfaces (16a, 25) are arranged such that, in this proximal blocking position, a space exists between them, the thickness of which is about 10 to 40% of the diameter of a suture thread (100).

4. The anchor (1) according to claim 1, **characterized in that** the positioning means are made up of two diametrically opposite longitudinal slots (18) arranged in said outer part (2) and corresponding ribs (27) arranged on the inner part (3), these ribs (27) forming pads intended to be received in the guideway formed by the slots (18).

5. The anchor (1) according to one of claims 1 to 4, **characterized in that** the outer part (2) has two diametrically opposite longitudinal apertures (17), arranged across from the openings of said transverse conduit (21) and extending over the entire movement of this conduit (21), from said distal nonblocking position to said proximal blocking position.

6. The anchor (1) according to claim 8, **characterized in that** the inner part (3) has two snapping protrusions (26) able to snap behind the edges of the outer part (2) delimiting the distal ends of said apertures (17).

7. The anchor (1) according to one of claims 1-9, **characterized in that** said inner part (3) has a pointed distal portion (23), protruding past the outer part (2), on the distal side, when said inner part (3) is in said distal nonblocking position.
